# FASCICULE DE BREVET EUROPEEN

(11) **EP 1 633 243 B1**
(45) Date de publication et mention de la délivrance du brevet: **04.04.2007**
(21) Numéro de dépôt: 04767277.9
(22) Date de dépôt: 07.06.2004
(51) Int. Cl.: A61B 5/00

(54) **PROCEDE ET DISPOSITIF D'ACQUISITION ET DE TRAITEMENT D'IMAGES D'UN OBJET TEL QU'UNE DENT**
VERFAHREN UND VORRICHTUNG ZUR AUFZEICHNUNG UND VERARBEITUNG VON BILDERN EINES OBJEKTS, WIE Z.B. EINES ZAHNS
METHOD AND DEVICE FOR RECORDING AND PROCESSING IMAGES OF AN OBJECT SUCH AS A TOOTH

(30) Priorité: 17.06.2003 FR 0307294
(43) Date de publication de la demande: 15.03.2006
(73) Titulaire: Centre National De La Recherche Scientifique-CNRS, 75794 Paris Cedex 16 (FR); UNIVERSITE DE BORDEAUX I, F-33405 Talence Cedex (FR)
(72) Inventeur: JONUSAUSKAS, Gediminas, F-33400 Talence (FR); OBERLE, Jean, F-33800 Bordeaux (FR); ABRAHAM, Emmanuel, F-33850 Leognan (FR)
(74) Mandataire: Ramey, Daniel
(86) Numéro de dépôt international: PCT/FR2004/001411
(87) Numéro de publication internationale: WO 2005/002429

(56) Documents cités:
- WO-A-01/41632
- WO-A-02/096281
- WO-A-03/005892
- DE-A- 4 200 741
- DE-U- 9 317 984
- US-B1- 6 293 911
- US-B1- 6 525 819
- US-E- R E31 815

## Description

L'invention concerne un procédé et un dispositif d'acquisition et de traitement d'images d'un objet, tel qu'une dent en bouche, éclairé en lumière ultraviolette.

On connaît par le document WO-A-02/096281 un procédé de détection précoce de caries dentaires, qui consiste à éclairer une dent en bouche avec une lumière ultraviolette ayant une longueur d'onde comprise entre 300 et 370 nm environ pour exciter une émission de fluorescence par la partie minérale de la dent, à prendre des images vidéo de la dent dans deux bandes de longueurs d'ondes de haute énergie et de basse énergie respectivement du spectre d'émission, à mesurer l'intensité spectrale de la fluorescence émise dans ces deux bandes en chaque point de l'image de la dent, à faire les rapports des intensités mesurées dans les deux bandes en chaque point de l'image et à comparer ces rapports à une valeur prédéterminée.

Le rapport des intensités mesurées dans une bande de longueurs d'ondes comprises entre la longueur d'onde d'excitation et 600 nm environ et dans une bande de longueurs d'ondes comprises entre 550 et 800 nm environ, indique la présence d'une carie quand il est inférieur à une valeur prédéterminée qui dépend des sensibilités des photo-détecteurs des moyens vidéo de prise d'image dans ces deux bandes de longueurs d'ondes.

Le dispositif utilisé pour l'exécution de ce procédé comprend un laser et des filtres pour l'éclairage de la dent par des impulsions successives de lumière ultraviolette et de lumière visible, et en ce qui concerne les moyens de prise d'image, un dispositif de filtrage spectral comprenant deux filtres, dont l'un transmet les signaux de la bande de longueurs d'onde de haute énergie et l'autre les signaux de la bande de longueurs d'onde de basse énergie, et une caméra vidéo qui reçoit successivement les signaux transmis dans la bande de haute énergie, les signaux transmis dans la bande de basse énergie et des signaux correspondant à l'image de la dent éclairée en lumière visible. Des moyens de traitement de l'information calculent les rapports précités en chaque point de l'image de la dent à partir des signaux de sortie de la caméra et affichent sur un écran une image de fluorescence et une image en lumière visible de la dent, ce qui permet de localiser avec précision les caries éventuelles sur l'image de la dent.

Ce dispositif connu fonctionne de façon satisfaisante mais est relativement coûteux. Il est aussi relativement peu rapide, puisqu'il faut interposer successivement des filtres devant la caméra pour acquérir les images de fluorescence dans les deux bandes de longueurs d'ondes et les images de la dent en lumière visible.

La présente invention a notamment pour but de pallier ces inconvénients.

Elle a pour objet un procédé et un dispositif du type précité, qui sont applicables notamment à la détection précoce des caries dentaires et de façon plus générale à la détection d'altérations et de variations des propriétés des surfaces et des volumes accessibles optiquement d'objets quelconques, ce procédé et ce dispositif utilisant des matériels et des composants simples et bon marché.

Elle a également pour objet un procédé et un dispositif du type précité, qui permettent une acquisition et un traitement plus rapides et plus précis des images de l'objet examiné.

Elle propose, à cet effet, un procédé d'acquisition et de traitement d'image d'un objet tel par exemple qu'une dent en bouche, consistant à éclairer l'objet en lumière ultraviolette, à prendre avec des moyens vidéo des images de la partie éclairée de l'objet, à mesurer en chaque point de ces images l'intensité spectrale de la luminescence émise par l'objet dans deux bandes de longueurs d'ondes de haute et de basse énergie respectivement, à faire le rapport de ces mesures en chaque point de l'image de l'objet et à comparer ce rapport à une valeur prédéterminée, caractérisé en ce qu'il consiste :
- à prendre au moins une image vidéo en couleurs de ladite partie de l'objet éclairée en lumière ambiante,
- à prendre au moins une image vidéo en couleurs de la luminescence produite par ladite partie de l'objet en réponse à l'éclairage de cette partie en lumière ultraviolette,
- à soustraire l'image vidéo en lumière ambiante de l'image vidéo de luminescence, en chaque point de cette image de luminescence, pour obtenir une image pure,
- à extraire les composantes spectrales de l'image pure dans lesdites bandes de haute et de basse énergie,
- à faire les rapports de ces composantes en chaque point d'image et à comparer ces rapports à une valeur prédéterminée.

Il suffit donc, dans le procédé selon l'invention, d'un éclairage de l'objet en lumière ambiante et d'un éclairage de l'objet en lumière ultraviolette pour obtenir au moyen d'une caméra vidéo couleur les informations nécessaires au calcul des rapports précités, et l'on n'a plus besoin d'utiliser des filtres que l'on commute devant la caméra vidéo, puisque l'on peut disposer directement des composantes des images vidéo dans les bandes de longueurs d'ondes de haute et de basse énergie.

Avantageusement, le procédé selon l'invention consiste à utiliser une caméra vidéo couleur du type rouge - vert - bleu pour prendre les images de l'objet et à extraire les composantes rouges et bleues des images vidéo pour calculer les rapports précités.

L'invention propose également un dispositif pour l'exécution de ce procédé, comprenant une source de lumière ultraviolette pour l'éclairage de l'objet, des moyens vidéo de prise d'images d'une partie éclairée de l'objet et des moyens de traitement de l'information recevant les signaux de sortie des moyens vidéo, caractérisé en ce que les moyens vidéo comprennent une caméra vidéo couleur et en ce que les moyens de traitement de l'information sont prévus pour soustraire, point par point, une image de la partie de l'objet éclairée en lumière ambiante d'une image de la partie de l'objet éclairée en lumière ultraviolette, afin d'obtenir une image pure, extraire les composantes de cette image pure dans deux bandes de longueurs d'ondes de haute énergie et de basse énergie respectivement, faire les rapports point par point de ces deux composantes et les comparer à une valeur prédéterminée.

Avantageusement, la caméra vidéo couleur utilisée est une caméra matricielle CCD du type "webcam" ayant un prix relativement très bas (typiquement de moins de 100 euros).

Selon d'autres caractéristiques de l'invention, ce dispositif comprend des moyens d'affichage d'un résultat formé par les points d'image dont les rapports sont inférieurs (ou supérieurs, respectivement) à la valeur prédéterminée.

La source de lumière ultraviolette utilisée dans ce dispositif est de préférence une diode électroluminescente émettant une lumière ultraviolette dans une bande étroite de longueurs d'onde centrée sur 370 nm environ par exemple.

Un dispositif comprenant ces moyens a un prix de revient très faible et est cependant d'une fiabilité et d'une précision surprenantes.

Dans un mode de réalisation préféré de l'invention, la caméra vidéo couleur, comprenant un objectif de formation d'images, et la source de lumière ultraviolette sont logées dans un boîtier de faibles dimensions et sont reliées par un câble souple aux moyens de traitement de l'information.

Ce boîtier est manipulable comme un endoscope et permet facilement des prises d'images sur les dents en bouche.

Si nécessaire, il peut contenir également une source de lumière visible pour l'éclairage de l'objet, lorsque l'éclairage ambiant n'est pas suffisant ou que la sensibilité des photo-détecteurs de la caméra est un peu faible.

De façon générale, l'invention est applicable à la détection d'altérations et de variations des propriétés des surfaces, des interfaces et des volumes accessibles optiquement d'un objet quelconque dans des domaines divers tels que l'oncologie, la biologie, la géologie, l'écologie, le contrôle industriel, etc.

L'invention sera mieux comprise, et d'autres caractéristiques, détails et avantages de celle-ci apparaîtront plus clairement à la lecture de la description qui suit, faite à titre d'exemple en référence aux dessins annexés dans lesquels :
- La figure 1 est une vue schématique du dispositif selon l'invention;
- La figure 2 est un organigramme des principales étapes du procédé selon l'invention;
- La figure 3 représente schématiquement une image de fluorescence d'une dent en bouche ;
- La figure 4 est un graphe représentant les variations des rapports de luminescence dans les bandes spectrales de haute et de basse énergie, le long de deux lignes de l'image de la figure 3.

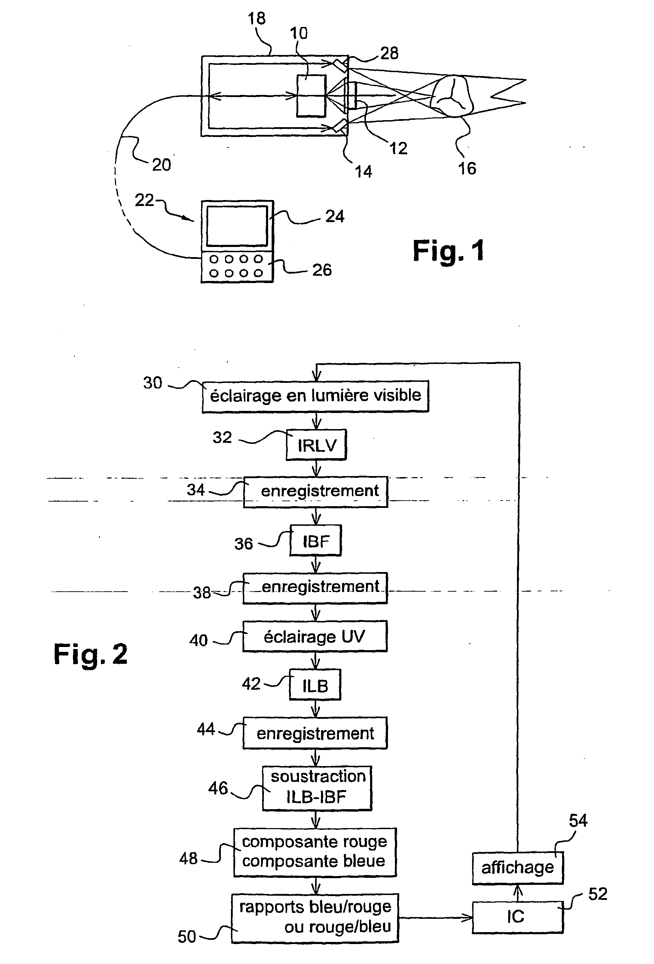

Le dispositif selon l'invention qui est représenté schématiquement en figure 1, comprend essentiellement des moyens de prise d'image vidéo couleur qui sont constitués d'un ensemble matriciel 10 de photo-détecteurs, tels que des photodiodes ou des photo-détecteurs CCD, et d'un objectif 12 de formation d'image sur l'ensemble 10 de photo-détecteurs, ainsi qu'une source 14 de lumière ultraviolette pour l'éclairage d'un objet 16; tel par exemple qu'une dent en bouche, situé devant l'objectif 12.

Dans un mode de réalisation préféré de l'invention, l'ensemble 10 de photo-détecteurs et l'objectif 12 sont ceux d'une caméra vidéo couleur du type "webcam", comprenant par exemple une matrice de 320 x 240 pixels d'une taille de ¼ de pouce (4 mm en diagonale) et un objectif de longueur focale de 5 mm, qui est disponible sur le marché à un prix relativement très faible.

La source de lumière ultraviolette est avantageusement une diode électroluminescente émettant dans une bande étroite de longueurs d'onde centrée sur 370 nm environ.

La caméra 10, 12 et la source 14 de lumière ultraviolette sont montées à l'intérieur d'un boîtier 18 par exemple cylindrique, ayant de faibles dimensions, du type endoscope par exemple qui est facilement manipulable d'une main ou qui est, en variante, monté de façon orientable sur un bras articulé de suspension ou de support.

Un câble souple 20 relie les composants 10, 14 logés dans le boîtier 18 à un appareil de commande 22 comprenant des moyens de traitement de l'information, un écran d'affichage 24 et un clavier de commande 26, ainsi que des moyens d'alimentation électrique de l'ensemble du dispositif selon l'invention.

Une source lumineuse 28 d'un type quelconque approprié peut également être logée dans le boîtier 18 pour l'éclairage de l'objet 16 en lumière visible, de préférence en lumière blanche.

Ce dispositif est utilisé de la façon suivante:

On positionne de façon correcte le boîtier 18 par rapport à l'objet 16, de façon à pouvoir éclairer cet objet en lumière ultraviolette et éventuellement-en lumière visible et à former une image nette de cet objet sur l'ensemble 10 de photo-détecteurs de la caméra.

La première étape du procédé selon l'invention, comprend, si nécessaire, l'éclairage 30 de l'objet 16 en lumière visible au moyen de la source 28, la prise 32 d'une image vidéo en couleurs ou en niveaux de gris de l'objet 16, cette image étant une image de référence en lumière visible IRLV, l'enregistrement 34 de cette image dans une mémoire de l'appareil 22, et l'extinction de la source 28 de lumière visible.

L'étape suivante du procédé comprend une prise 36 d'image en couleurs de l'objet 16 éclairé en lumière ambiante, pour obtenir une image de bruit de fond IBF, et l'enregistrement 38 de cette image dans la mémoire de l'appareil 22.

L'étape suivante du procédé comprend l'éclairage 40 de l'objet 16 en lumière ultraviolette au moyen de la source 14, la prise 40 d'une image vidéo en couleurs de l'objet 12, cette image étant une image de luminescence brute ILB, l'enregistrement 44 de cette image dans la mémoire de l'appareil 22, et l'extinction de la source 14 de lumière ultraviolette.

L'éclairage en lumière ultraviolette de l'objet 16 est réalisé par des impulsions d'une durée correspondant au temps d'exposition de la caméra CCD, chaque impulsion provoquant une fluorescence de la partie minérale de la dent et les impulsions d'éclairage étant séparées l'une de l'autre par un intervalle de temps adapté aux séquences d'acquisition des trames vides.

Pour améliorer la précision et réduire le bruit, on peut accumuler plusieurs trames vidéo correspondant aux images de référence en lumière visible IRLV, de bruit de fond IBF et de luminescence brute ILB et les moyenner, le nombre de trames accumulées étant le même pour chaque type d'image.

Les étapes suivantes du procédé comprennent:
- En 46, une soustraction point par point (pixel par pixel ou groupe de pixels par groupe de pixels) de l'image en lumière ambiante IBF de l'image en éclairage ultraviolet ILB pour obtenir une image IL de luminescence pure,
- En 48, l'extraction des composantes rouge ILr et bleue ILb de l'image IL de luminescence pure,
- En 50, le calcul point par point des rapports ILb/ILr (ou ILr/ILb),
- En 52, la comparaison de ces rapports à une valeur prédéterminée,
- En 54, l'affichage d'une image de carie IC formée des points pour lesquels les rapports précités ILb/ILr sont inférieurs à une valeur prédéterminée (ou des points pour lesquels les rapports ILr/ILb sont supérieurs à une valeur prédéterminée).

Avantageusement, on affiche simultanément sur l'écran 24 l'image de carie IC en fausses couleurs et l'image de la dent éclairée en lumière visible ou en lumière ambiante. Cela permet de repérer facilement avec précision les zones de carie sur la dent examinée.

Ensuite, l'appareil de commande redémarre un cycle d'acquisition et de mesure au début, c'est-à-dire à l'étape 30.

L'éclairage en lumière visible de l'objet examiné ne présente un intérêt que si l'image vidéo de cet objet en lumière ambiante n'est pas suffisamment bonne.

Dans la plupart des cas, la-sensibilité des photo-détecteurs de la caméra vidéo permet de se dispenser de l'éclairage de l'objet en lumière visible au moyen de la source 28.

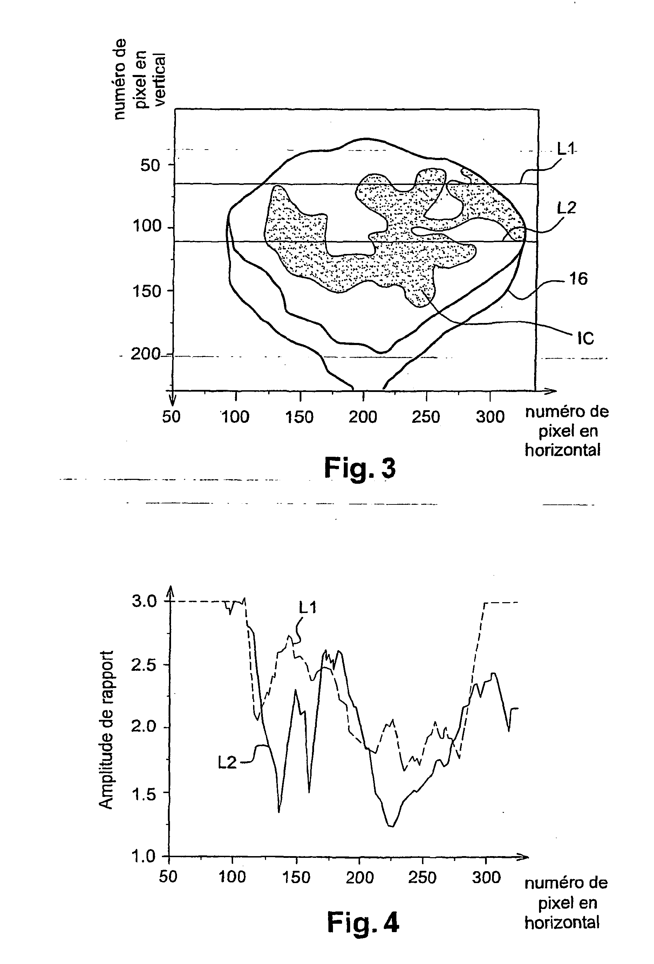

La figure 3 représente schématiquement une image de carie IC qui est affichée en fausses couleurs, par exemple en rouge ou en bleu, éventuellement sur une image d'une dent éclairée en lumière visible IRLV ou en lumière ambiante IBF, les abscisses désignant les numéros des pixels dans les rangées horizontales de l'ensemble de photo-détecteurs de la caméra, les ordonnées désignant les numéros des pixels dans les colonnes verticales des photo-détecteurs de cet ensemble matriciel.

La figure 4 représente, pour deux lignes de mesures L1 et L2 de l'image de la figure 3, les variations du rapport ILb/ILr en fonction des numéros des pixels dans les rangées horizontales précitées.

Les valeurs de ce rapport qui sont voisines de 2,5 correspondent à un émail sain et celles qui sont inférieures à 2 correspondent à une carie.

Un certain nombre de variantes peuvent être apportées au dispositif selon l'invention qui a été décrit et représenté, sans sortir du cadre de l'invention défini par les revendications qui suivent.

Par exemple, le capteur vidéo couleur 10 du dispositif selon l'invention peut être un capteur matriciel du type CCD ou un capteur matriciel à photodiodes, ou un capteur matriciel du type CMOS, ou encore un vidicon couleur, ou tout autre dispositif matriciel d'acquisition d'image couleur ou de séquence vidéo en couleur.

Les moyens de formation d'image sur ce capteur vidéo peuvent être formés d'un objectif, de lentilles, de miroirs, de guides d'image à fibres optiques, etc..., et en général de tout moyen permettant de former une image d'un objet 16 sur le capteur vidéo 10.

La source 14 d'éclairage en lumière ultraviolette peut être une diode électroluminescente comme déjà indiqué, un autre composant semi-conducteur, une source thermique, une lampe à décharge électrique, un laser, etc...., produisant une lumière ultraviolette dans la bande de longueurs d'onde de 300 à 370 nm environ permettant d'exciter la luminescence de la partie minérale de la dent 16.

La lumière émise par cette source n'est soumise à aucun critère de monochromaticité, de cohérence ou d'état de polarisation. Bien entendu, cette source de lumière peut être associée à des éléments optiques de collimation, de transmission et / ou d'atténuation de puissance du faisceau lumineux émis.

De même, la source 28 de lumière visible peut être une diode électroluminescente, un autre composant semi-conducteur, une source thermique, une lampe à décharge électrique, un laser, etc..., et en général tout moyen permettant de générer une lumière visible, de préférence blanche pour l'éclairage de l'objet 16.

Cette source de lumière 28 peut être associée à des moyens de collimation, de transmission et / ou d'atténuation de puissance du faisceau lumineux émis.

Ces sources -lumineuses 14, 28 peuvent être des sources ponctuelles ou étendues, uniques ou multiples, et sont assemblées sur le boîtier 18 à une extrémité de celui-ci ou sont éloignées, la lumière étant transmise par des fibres optiques par exemple.

## Revendications

1. Procédé d'acquisition et de traitement d'images d'un objet, tel qu'une dent en bouche, consistant à éclairer l'objet (16) en lumière ultraviolette, à prendre avec des moyens vidéo (10,12) des images de la partie éclairée de l'objet, à mesurer en chaque point de ces images l'intensité spectrale de la luminescence émise par l'objet dans deux bandes de longueurs d'ondes de haute et de basse énergie respectivement, à faire le rapport de ces mesures en chaque point de l'image de l'objet et à comparer ces rapports à une valeur prédéterminée, consistant :
- à prendre au moins une image vidéo en couleurs de ladite partie de l'objet (16) éclairée en lumière ambiante,
- à prendre au moins une image vidéo en couleurs de la luminescence produite par ladite partie de l'objet (16) en réponse à son éclairage en lumière ultraviolette,
**caractérisé en ce qu'**il consiste, de plus :
- à soustraire l'image vidéo-en lumière ambiante de l'image vidéo de luminescence, en chaque point de cette image de luminescence, pour obtenir une image pure,
- à extraire les composantes spectrales de l'image pure dans lesdites bandes de longueurs d'ondes de haute et de basse énergie,
- à faire les rapports de ces composantes en chaque point d'image et à comparer ces rapports à une valeur prédéterminée.

2. Procédé selon la revendication 1, **caractérisé en ce qu'**il consiste à afficher sur un écran un résultat formé des points d'image pour lesquels le rapport précité est inférieur (ou supérieur, respectivement) à la valeur prédéterminée précitée.

3. Procédé selon la revendication 2, **caractérisé en ce qu'**il consiste à afficher simultanément ledit résultat et ladite image vidéo en couleurs de la partie de l'objet éclairée en lumière ambiante ou une image vidéo de cette partie de l'objet éclairée en lumière visible.

4. Procédé selon la revendication 3, **caractérisé en ce qu'**il consiste à afficher le résultat précité en fausses couleurs sur l'image vidéo de l'objet en lumière ambiante ou en lumière visible.

5. Procédé selon l'une des revendications précédentes, **caractérisé en ce qu'**il consiste à prendre des images de l'objet au moyen d'une caméra matricielle vidéo du type rouge - vert - bleu,- et à utiliser les composantes rouge et bleue de ladite image pour calculer les rapports précités.

6. Procédé selon l'une des revendications précédentes, **caractérisé en ce qu'**il consiste à prendre plusieurs images vidéo de la partie de l'objet éclairée en lumière ambiante et en lumière ultraviolette, à calculer des moyennes de ces images, puis à utiliser ces moyennes pour calculer les rapports précités.

7. Dispositif pour l'exécution du procédé décrit dans l'une des revendications précédentes, comprenant une source (14) de lumière ultraviolette pour l'éclairage d'un objet (16), des moyens vidéo (10,12) de prise d'image d'une partie éclairée de l'objet et des moyens (22) de traitement de l'information recevant les signaux de sortie des moyens vidéo, **caractérisé en ce que** les moyens vidéo comprennent une caméra vidéo couleur (10,12) et **en ce que** les moyens de traitement de l'information sont prévus pour soustraire, point par point; une image de la partie de l'objet éclairée en lumière ambiante d'une image de la partie de l'objet éclairée en lumière ultraviolette, afin d'obtenir une image pure, extraire des composantes de cette image pure dans les deux bandes de longueurs d'ondes de haute et de basse énergie, faire les rapports point par point de ces deux composantes et les comparer à une valeur prédéterminée.

8. Dispositif selon la revendication 7, **caractérisé en ce qu'**il comprend des moyens (24) d'affichage du résultat formé par les points d'image dont les rapports sont inférieurs (ou supérieurs, respectivement) à la valeur prédéterminée.

9. Dispositif selon la revendication 7 ou 8, **caractérisé en ce qu'**il comprend des moyens (24) d'affichage d'une image en lumière ambiante ou d'une image en lumière visible de la ladite partie de l'objet et du résultat formé par les points d'image ayant des rapports inférieurs (ou supérieurs, respectivement) à la valeur prédéterminée.

10. Dispositif selon l'une des revendications 7 à 9, **caractérisé en ce que** la caméra vidéo couleur (10,12) est une caméra matricielle CCD, CMOS ou à photodiodes ou un vidicon couleur.

11. Dispositif selon l'une des revendications 7 à 10, **caractérisé en ce que** la source (14) de lumière ultraviolette est une diode électroluminescente émettant dans une bande de longueurs d'onde comprise entre 300 et 370 nm environ.

12. Dispositif selon l'une des revendications 7 à 11, **caractérisé en ce que** la caméra vidéo couleur, comprenant un objectif (12) de formation d'image, et la source (14) de lumière ultraviolette sont logées dans un boîtier (18) de faibles dimensions et sont reliées par un câble souple (20) aux moyens (22) de traitement de l'information.

13. Dispositif selon l'une des revendications 7 à -12, **caractérisé en ce que** le boîtier (18) contient également une source (28) de lumière visible pour l'éclairage de l'objet (16).

## Claims

1. A method of acquiring and processing images of an article, such as a tooth in the mouth, consisting in illuminating the article (16) in ultraviolet light, in using video means (10, 12) to take images of the illuminated portion of the article, in measuring at each point of said images the spectral intensity of the luminescence emitted by the article in two wavelength bands respectively at high energy and at low energy, in taking the ratio of said measurements at each point of the image of the article, and in comparing said ratios with a predetermined value, consisting:
· in taking at least one color video image of said portion of the article (16) illuminated in ambient light,
· in taking at least one color video image of the luminescence produced by said portion of the article (16) in response to being illuminated in ultraviolet light, **characterized in that** it further consists:
· in subtracting the ambient light video image from the luminescence video image at each point of the luminescence image in order to obtain a pure image,
· in extracting the spectral components of the pure image in said high-energy and low-energy wavelength bands, and
· in taking the ratios of said components at each point of the image and in comparing said ratios with a predetermined value.

2. A method according to claim 1, **characterized in that** it consists in displaying on a screen a result made up of image points for which the above-mentioned ratio is less than (or respectively greater than) the above-mentioned predetermined value.

3. A method according to claim 2, **characterized in that** it consists in displaying said result simultaneously with said color video image of the portion of the article as illuminated in ambient light or a video image of said portion of the article as illuminated in visible light.

4. A method according to claim 3, **characterized in that** it consists in displaying the above-mentioned result in false colors superimposed on the video image of the article in ambient light or in visible light.

5. A method according to any preceding claim, **characterized in that** it consists in taking images of the article by means of a red-green-blue type matrix video camera, and in using the red and blue components of said image to calculate the above-mentioned ratios.

6. A method according to any preceding claim, **characterized in that** it consists in taking a plurality of video images of the portion of the article illuminated in ambient light and in ultraviolet light, in calculating the averages of said images, and then in using said averages for calculating the above-mentioned ratios.

7. Apparatus for performing the method described in any preceding claim, comprising an ultraviolet light source (14) for illuminating an article (16), video means (10, 12) for taking an image of the illuminated portion of the article, and data processor means (22) receiving the output signals from the video means, **characterized in that** the video means comprise a color video camera (10, 12), and **in that** the data processor means are designed to subtract, point by point, an image of the portion of the article illuminated in ambient light from an image of the portion of the article illuminated in ultraviolet light so as to obtain a pure image, to extract the components from said pure image in the two wavelength bands respectively at high energy and at low energy, to take the ratios point by point of these two components, and to compare them with a predetermined value.

8. Apparatus according to claim 7, **characterized in that** it comprises means (24) for displaying the results formed by the image points for which the ratios are less than (or respectively greater than) the predetermined value.

9. Apparatus according to claim 7 or claim 8, **characterized in that** it includes means (24) for displaying an ambient light image or a visible light image of said portion of the article and the result formed by the points of the image having ratios that are less than (or respectively greater than) the predetermined value.

10. Apparatus according to any one of claims 7 to 9, **characterized in that** the color video camera (10, 12) is a CCD, CMOS, or photodiode matrix camera, or a color vidicon.

11. Apparatus according to any one of claims 7 to 10, **characterized in that** the ultraviolet light source (14) is an LED emitting in a wavelength band lying in the range about 300 nm to about 370 nm.

12. Apparatus according to any one of claims 7 to 11, **characterized in that** both the ultraviolet light source (14) and the color video camera including an image-forming lens (12) are housed in a housing (18) of small dimensions, and are connected by a flexible cable (20) to the data processor means (22).

13. Apparatus according to any one of claims 7 to 12, **characterized in that** the housing (18) also contains a source (28) of visible light for illuminating the article (16).

## Patentansprüche

1. Verfahren zur Erfassung und Bearbeitung von Bildern eines Objektes, wie einem Zahn, bestehend aus der Ausleuchtung des Objektes (16) mit ultraviolettem Licht, dem Aufnehmen von Bildern des beleuchteten Teiles des Objektes durch Videoeinrichtungen (10, 12), dem Messen der Spektralintensität der von dem Objekt in zwei Wellenlängenbanden emittierten Lumineszenz hoher bzw. niedriger Energie in jedem Punkt dieser Bilder, dem Bilden eines Quotienten dieser Messungen an jedem Punkt des Bildes des Objektes und dem Vergleichen der Quotienten mit einem vorbestimmten Wert, bestehend aus:
- dem Aufnehmen wenigstens eines Farbvideobildes des Teiles des mit Umgebungslicht ausgeleuchteten Gegenstandes (16),
- dem Aufnehmen wenigstens eines Farbvideobildes der Lumineszenz, welche durch den Teil des Gegenstandes (16) in Antwort auf seine Ausleuchtung mit ultraviolettem Licht erzeugt wird, **dadurch gekennzeichnet, dass** es des Weiteren besteht aus
- dem Subtrahieren des Umgebungslichtvideobildes von dem Lumineszenzvideobild in jedem Punkt des Lumineszenzbildes, um ein reines Bild zu erhalten,
- dem Extrahieren der Spektralbestandteile des reinen Bildes in den Bändern der Wellenlängen hoher und niedriger Energie,
- dem Bilden der Quotienten dieser Bestandteile in jedem Punkt des Bildes und dem Vergleichen dieser Quotienten mit einem vorbestimmten Wert.

2. Verfahren nach Anspruch 1, **dadurch gekennzeichnet, dass** es aus dem Anzeigen eines Resultates, das aus Bildpunkten, für welche der vorgenannte Quotient unter (bzw. über) dem vorgenannten vorbestimmten Wert liegt, auf einem Bildschirm besteht.

3. Verfahren nach Anspruch 2, **dadurch gekennzeichnet, dass** es aus der gleichzeitigen Anzeige des Resultates und des Farbvideobildes des Teiles des mit Umgebungslicht ausgeleuchteten Gegenstandes oder einem Videobild dieses Teiles des mit sichtbarem Licht ausgeleuchteten Gegenstandes besteht.

4. Verfahren nach Anspruch 3, **dadurch gekennzeichnet, dass** es aus dem Anzeigen des vorgenannten Resultates als Fehlfarben auf dem Videobild des Gegenstandes unter Umgebungslicht oder sichtbarem Licht besteht.

5. Verfahren gemäß einem der vorhergehenden Ansprüche, **dadurch gekennzeichnet, dass** es im Aufnehmen von Bildern des Objektes mittels einer Videomatrixkamera vom Typ Rot-Grün-Blau und in der Verwendung der roten und blauen Bestandteile des Bildes zur Berechnung der vorgenannten Quotienten besteht.

6. Verfahren gemäß einem der vorhergehenden Ansprüche, **dadurch gekennzeichnet, dass** es darin besteht, mehrere Videoaufnahmen des Teiles des mit Umgebungslicht und Ultraviolettlicht ausgeleuchteten Gegenstandes aufzunehmen, den Mittelwert dieser Bilder zu berechnen und dann diese Mittelwerte zur Berechnung der vorgenannten Quotienten zu verwenden.

7. Vorrichtung zur Durchführung des in den vorhergehenden Ansprüchen beschriebenen Verfahrens, umfassend eine Quelle (14) für ultraviolettes Licht zur Ausleuchtung eines Gegenstands (16), Videoeinrichtungen (10, 12) zur Aufnahme von Bildern eines ausgeleuchteten Teiles des Objektes und Mittel (22) zur Verarbeitung der Informationen, welche die Signale vom Ausgang der Videoeinrichtungen empfangen, **dadurch gekennzeichnet, dass** die Videoeinrichtungen eine Farbvideokamera (10, 12) umfassen und **dadurch**, dass die Mittel zur Verarbeitung der Information vorgesehen sind, um Punkt-für-Punkt ein Bild des Teiles des mit Umgebungslicht ausgeleuchteten Gegenstandes von einem Bild des Teiles des mit Ultraviolettlicht ausgeleuchteten Gegenstandes zu subtrahieren, um ein reines Bild zu erhalten, die Bestandteile dieses reinen Bildes in den zwei Bändern der Wellenlängen hoher und niedriger Energie zu extrahieren, den Quotienten dieser zwei Bestandteile Punkt-für-Punkt zu erstellen und mit einem vorbestimmten Wert zu vergleichen.

8. Vorrichtung nach Anspruch 7, **dadurch gekennzeichnet, dass** sie Mittel (24) zum Anzeigen der Resultate umfasst, welche durch die Punkte des Bildes gebildet sind, deren Quotienten unter (oder bzw. über) einem vorbestimmten Wert liegen.

9. Vorrichtung nach Anspruch 7 oder 8, **dadurch gekennzeichnet, dass** sie Mittel (24) zum Anzeigen eines Umgebungslichtbildes oder eines Bildes von sichtbarem Licht des Teiles des Gegenstandes und des Ergebnisses, welches durch die Punkte des Bildes gebildet sind, welche niedrigere (bzw. höhere) Quotienten im Vergleich zu dem vorbestimmten Wert aufweisen, umfasst.

10. Vorrichtung gemäß einem der Ansprüche 7 bis 9, **dadurch gekennzeichnet, dass** die Farbvideokamera (10, 12) eine CCD-, CMOS-Matrixkamera oder eine solche mit Fotodioden oder eine Farb-Vidikonkamera ist.

11. Vorrichtung nach einem der Ansprüche 7 bis 10, **dadurch gekennzeichnet, dass** die Quelle (14) für ultraviolettes Licht eine Elektrolumineszenzdiode ist, welche ein Wellenlängenband emittiert, das zwischen etwa 300 und 370 nm enthalten ist.

12. Vorrichtung gemäß den Ansprüchen 7 bis 11, **dadurch gekennzeichnet, dass** die Farbvideokamera, welche ein Objektiv (12) zur Bildung eines Bildes umfasst und die Quelle (14) für ultraviolettes Licht in einem Gehäuse (18) geringer Abmessungen untergebracht sind und durch ein biegsames Kabel (20) mit den Mitteln (22) zur Verarbeitung der Informationen verbunden sind.

13. Vorrichtung nach einem der Ansprüche 7 bis 12, **dadurch gekennzeichnet, dass** das Gehäuse (18) des Weiteren eine Quelle (28) für sichtbares Licht zum Ausleuchten des Gegenstandes (16) enthält.
